Europäisches Patentamt

European Patent Office

Office européen des brevets

(11) Publication number: **0 282 746**
**A1**

# EUROPEAN PATENT APPLICATION

(21) Application number: 88102250.3

(51) Int. Cl.⁴: **C12N 5/00**

(22) Date of filing: 17.02.88

(30) Priority: 19.02.87 JP 37585/87

(43) Date of publication of application:
21.09.88 Bulletin 88/38

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Hata, Ryu-ichiro
7-17, Ohanaminami 1-chome
Funabashi Chiba 274(JP)

(74) Representative: von Kreisler, Alek,
Dipl.-Chem. et al
Deichmannhaus am Hauptbahnhof
D-5000 Köln 1(DE)

(54) Artificial cultured tissue and its production.

(57) The present invention relates to an artificial tissue comprising cells and extracellular matrix produced by growing a connective tissue cell in an ascorbic acid phosphate-containing medium.

The artificial tissue of the present invention can be utilized as an artificial skin, artificial blood vessel, artificial bone, artificial liver or the like.

## Artificial Cultured Tissue and Its Production

The present invention relates to an artificial tissue and a method of preparing the same.

It is known that various cells in the course of differentiation synthesize tissue-specific extracellular matrix (ECM) substances such as collagen, proteoglycan, etc. and that these ECM substances in turn play important roles in the attachment, the proliferation and differentiation of such cells. Collagen, which is a main constituent of ECM, accounts for 30% of the protein in higher animals.

Collagen, products of about 20 gene groups for subunits ($\alpha$ chains), occurs in 11 different types and the distribution of them show cell or tissue specificity. In the course of differentiation, cells synthesize various types of collagen, which promote cell differentiation by providing a micro environment and are involved in the maintenance of tissue homeostasis.

As examples of the role collagen plays as a factor in differentiation. There have been reported on the differentiation from myoblast to myotube, from somite to cartilage, or differentiation of ocular tissues.

Because collagen is a main constituent of the ECM produced by mesenchymal cells such as fibroblasts in the connective tissues such as the skin tissue, its application to the artificial skin has heretofore been attempted.

When a burn or an accident results in an extensive skin defect, the victim's life is seriously endangered. In the treatment of such a skin defect, it is important to cover up the defected skin area with a skin prosthesis to prevent loses of body fluid and infection from the wound surface in addition to systemic management. As the material for such skin substitutes, there may be contemplated (1) a processed animal skin, (2) a human skin other than the patient's, (3) the patient's, own skin, and (4) an artificial skin composed of a synthetic high polymer or collagen. However, each of these materials has its own disadvantages. For example, in the case of (1) and (2), the patient's rejection reaction is clinically a problem. The material (3) is ideal but has a limitation on availability. The material (4) is intended to compensate for shortcomings of (1) through (3) and while the following and other contrivances have been introduced, none have proved fully satisfactory.

Thus, fibroblasts are collected from a small specimen of the patient's skin and cultured and the resultant culture is mixed with collagen to give a collagen gel containing fibroblasts. As this gel is incubated at 37°C, the collagen fibers shrink to give a dermis (corium)-like sheet. Using this sheet as a substitute for the actual corium layer of the skin, that is to say a pseudocorium layer, epidermal cells separated from the patient are inoculated over the layer and cultured, whereupon the epidermal cells multiply to form an epidermis-like structure, thus yielding a artificial skin.

The above pseudocorium is quite different from the genuine corium and is therefore deficient in the property of the repaired wound surface, for instance, and there has accordingly been a standing demand for a prosthetic tissue more closely similar to the biological skin.

Regarding the application of collagen, it is also known that when epithelial cells of the mammary gland are cultured on a collagen gel suspension, the synthesis and secretion of casein takes place.

Furthermore, in culturing capillary vascular endothelial cells, pretreatment of culture dishes with collagen allows the cells to form a tubular structure by themselves as a manifestation of their own inherent nature.

It is also known that in the presence of a bone matrix consisting predominantly of collagen, immature mesenchymal cells in muscle tissue differentiated into chondrocytes.

Recently, it has become clear that epithelial cells such as hepatic parenchymal cells also produce collagen, as a departure from the earlier common belief. Cultured hepatocytes usually die within 1 to 2 weeks but it is by now known that when grown in the presence of the collagen-containing intercellular material, hepatocytes can be kept viable for a fairly long time without interfering with their normal function.

Thus, many applications of collagen can be contemplated, not only as an artificial skin but also for the formation of a variety of tissues and organs. However, collagen is nothing but a predominant constituent of ECM and, moreover, occurs in a variety of molecular species. Therefore, to provide an ideal artificial tissue, it is desirable to develop a method for stimulating the formation of a tissue closely resembling the biological tissue, for example, a connective tissue, through the use of the very cells constituting the tissue and through a cultural procedure which would stimulate the growth and differentiation of these cells.

Ascorbic acid has long been known to be essential to the living body as antiscorbutic factor or vitamin C. It is also known that ascorbic acid has a variety of activities [Annals of the New York Academy of Sciences 258, 1975] and particularly it has been shown that this substance is a cofactor of proline hydroxylase which plays a key role in collagen synthesis [Proceedings of the National Academy of Sciences of the United States of America (hereinafter referred to sometimes briefly as Proc. Natl. Acad. Sci. U.S.A.) 53, 335, 1965]

Recent years have witnessed that many researches were done using tissue culture technologies for the elucidation of mechanisms regulating metabolism. It has recently been suggested that ascorbic acid not only acts as a cofactor for the above-mentioned enzyme but also exerts various effects on cells. Among such actions are those related to the intensification of manifestation of hormone activities (1)* and the differentiation of fibroblasts (2, 3)*, smooth muscle cells (4)*, and chondrocytes (5)*.

*(1) Proc. Natl. Acad. Sci. U.S.A. 73, 183 (1976)

(2) Proc. Natl. Acad. Sci. U.S.A. 74, 4453 (1977)

(3) Journal of Investigative Dermatology), 79, 735 (1982)

(4) Journal of Cell Biology, 92, 462 (1982)

(5) Cell, 30, 215 (1982)

However, ascorbic acid is very labile under tissue culture conditions (for example, 37°C, in solution at neutral pH) and rather cytotoxic in high concentrations. Therefore, in conducting tissue culture, ascorbic acid must be added continuously. As a result, for proceeding with researches into the effects of ascorbic acid on the growth, differentiation, metabolism, etc. of connective tissue cells in culture, such as fibroblasts, smooth muscle cells, chondrocytes, vascular endothelial cells, etc. and further proceeding with the in vitro formation of a tissue close to the biological tissue for the application of such tissue as an artificial tissue, the development of an ascorbic acid derivative which would remain stable in culture medium has been demanded.

The term "artificial tissue" as used herein of course includes not only technologies related to the formation of artificial skins but also those related to other artifacts such as artificial blood vessel, artificial bone, artificial liver, and so on.

In view of the above situation, the present inventor explored the method of culturing cells including connective tissue cells for multiplication and found that if an ascorbic acid phosphate in lieu of ascorbic acid is added to the culture medium, this ascorbic acid derivative will not only remain stable in the medium but effectively promote cell proliferation, cell differentiation, and synthesis of an ECM having a composition close to that of the tissue in vivo.

Furthermore, the present inventor found that prolonged culture of connective tissue cells yields an excellent artificial tissue consisting of the connective tissue cells and ECM and, for this purpose, the addition of an ascorbic acid phosphate is very effective.

The present invention has been accomplished after further research based on the above findings.

The present invention is thus directed to a method of producing an artificial tissue which comprises growing a connective tissue cell in an ascorbic acid phosphate-containing medium to produce a tissue comprising connective tissue cells and ECM and recovering the same, and to an artificial tissue comprising connective tissue cells and ECM produced by growing a connective tissue cell in an ascorbic acid phosphate-containing medium.

As examples of connective tissue cell to be employed in the method of the invention, there may be mentioned fibroblasts, smooth muscle cells, chondrocytes, endothelial cells and so on. The fibroblasts include human dermal fibroblasts, human embryonic fibroblasts, rat dermal fibroblasts, chick embryonic tendon fibroblasts, and so on. The smooth muscle cells include smooth muscle cells of the human artery and those of the rat artery, for instance. The chondrocytes include, among others, human or rat epiphysial cartilage and chick embryonic epiphysial cartilage cells. The endothelial cells include, among others, bovine, porcine or human arterial endothelial cells, bovine brain endothelial cells, bovine or human venous endothelial cells, and bovine, human, rat or other capillary vascular endothelial cells.

The medium to be employed in the method of the invention may be any of the culture media that can be used in the culture of cells such as connective tissue cells, there being no particular limitation on types of medium.

Thus, Dulbecco's modified Eagle's minimum essential medium (DMEM) and other media for animal cell culture can be advantageously employed. Preferably incorporated in such media are animal serum, antibiotic, and so on. The animal serum is preferably fetal calf serum and is generally added at the level of about 0.1 to 50 percent (v/v) and preferably in the range of about 2 to 20 percent (v/v). The antibiotic may for example be kanamycin, penicillin, streptomycin or the like, and is generally added at the level of about 0.05 to 1 mg/ml.

The ascorbic acid phosphate to be incorporated in the medium in the method of the invention include ascorbic acid 2-phosphate, ascorbic acid 3-phosphate, ascorbic acid 2,3-diphosphate, etc. and a mixture of these two or three esters may be employed as well. Particularly preferred is ascorbic acid 2-phosphate.

The ascorbic acid phosphate may be used in the form of a salt. As such salt, the alkali metal salts (such as sodium salt, patassium salt, etc.) and alkaline earth metal salts (such as calcium salt, magnesium salt, etc.) may be mentioned, for instance.

The ascorbic acid phosphate mentioned above can be generally used as it is without any processing or formulation.

In the method of the invention, the ascorbic acid phosphate may be added to the medium in an early stage of culture in a logarithmic phase, or may be added after a stationary phase has been reached. Particularly where the formation of a tissue is intended, it is preferable to add the ester in an early stage of culture over a comparatively long time.

The amount of the ascorbic acid phosphate to be added to the medium, in terms of concentration in the medium, is about 0.01 to 5 mM and preferably about 0.05 to 0.4 mM. The addition may be made on one occasion only, or depending on the duration of culture, several times at appropriate intervals. It is particularly desirable to add about 0.2 mM of ascorbic acid phosphate to the medium at intervals of about 3 to 4 days when the medium is replaced with a fresh one.

The incubation temperature, medium pH, incubation time and other cultural conditions can be appropriately selected with reference to the properties of the cells to be grown. By way of example, the incubation temperature may be about 30 to 40°C and preferably about 35 to 38°C. The medium pH may for example be about 6.5 to 8 and preferably about 7 to 7.4. The incubation time may for example be 2 weeks or longer and preferably about 3 weeks to about 3 months. Under these conditions, a very satisfactory artificial tissue can be produced.

The cultural method that can be employed includes stationary culture, culture utilizing a microcarrier, or culture utilizing a hollow fiber device, for instance. For the purpose of accelerating cell differentiation, stationary culture is also one of preferred methods.

Dermal fibroblasts obtained by biopsy may be grown by the outgrowth method (the method in which a finely fragmented tissue is stuck on in a culture dish, a medium is then added, and fibroblasts are allowed to spread and multiply spontaneously in the dish owing to the cells' own motility).

For assessment of the degree of cell growth, the number of cells may be directly counted by hemocytometer or the DNA content instead of the number of cells may be determined. As an indirect method, a metabolic activity parameter such as RNA-or protein-synthetic activity may be determined. Particularly in the case of fibroblasts, since the uptake of proline in protein is correlated with collagen synthesis, the differentiation of a cell and particularly the degree of production of ECM can be estimated by determining the uptake of proline into protein or, to be more exact, the uptake activity which is digested with collagenase.

The resulting artificial tissue of the present invention occurs generally in the form of a sheet on the wall of the culture vessel. Therefore, it can be used as it is for purposes of an artificial tissue, or if desired, may be separated as a self-supporting sheet from the vessel wall with the aid of an exfoliating agent such as ethylenediamine tetracetate (EDTA) or the like.

It is known that collagen is synthesized as the procollagen in the cell and is processed to collagen. It is also known that collagen occurs in 11 different types, type I through type XI, and the distribution of these types shows cell or tissue specificity. Moreover, collagen molecules coalesce with each other to give collagen fiber which forms complexes with other extracellular matrix components [acidic glycosaminoglycan (GAG)], etc. to provide ECM.

Collagen can be distinguished from procollagen by utilizing their difference that whereas collagen is resistant to proteases other than collagenase, procollagen is processed by pepsin, etc. into collagen. Moreover, various types of collagen molecules can be identified by such techniques as SDS-polyacrylamide gel electrophoresis (SDS-PAGE). Further, for ascertaining the presence of a higher order structure of collagen which forms on crosslinking of collagen molecules, the collagen obtained by pepsin digestion is subjected to SDS-PAGE under reducing conditions and the $\beta$-(dimer) and $\gamma$-fractions (trimer) distributed on the higher molecular weight side of the collagen $\alpha$-chain (monomer) are determined.

By carrying out such analyses of collagen molecules, the degree of acceleration of cell differentiation in the culture system can be estimated and various comparisons of the artificial tissue with the biological tissue can be made.

To ascertain the synthesis of GAG which is a major ECM constituent as well as collagen, the molecular species and relative amounts of GAG formed can be determined, for example, by causing the cells to take up glucosamine, digesting them with Actinase E and subjecting them to two-dimensional electrophoresis on a cellulose acetate membrane. Then, the absolute amount of GAG can be estimated by the colorimetric determination of the GAG spot and the rate of synthesis thereof can be estimated by pulse labeling of the cells with the radiolabeled glucosamine.

By proceeding with such GAG analyses, too, the degree of acceleration of cell differentiation in the culture system can be estimated and various comparisons with the biological tissue can be made, as in the case of collagen analyses, from a different point of view.

For detailed investigation of cell differentiation, there is available a method of estimating the degrees of development of Golgi apparatus and rough endoplasmic reticulum. By such a procedure, too, the acceleration of cell differentiation in the culture system can be investigated.

By practicing the method of the invention in this manner, the growth and differentiation of connective tissue and other cells can be promoted and the formation of an artificial tissue or organ can be made feasible.

The use of an ascorbic acid phosphate in lieu of ascorbic acid in accordance with the present invention not only enables one to conduct researches into the proliferation, differentiation, metabolism, etc. of cultured cells, particularly through culture of various connective tissue cells with greater advantage but also enables one to reproduce in vitro a corium or dermis close to the biological dermal tissue, for instance, so that the product can be applied as a tissue prosthesis, e.g. an artificial skin, or an artificial organ. Particularly, when the method is applied using the patient's own cells, the rejection reaction at organ transplantation or the associated ethical problem can be obviated.

The artificial tissue according to the present invention closely resembles the animal dermis in the type of connective tissue contained and in the properties and composition of the ECM consisting of collagen, procollagen, GAG and so on. Therefore, it can be used advantageously as a prosthesis for the formation of an artificial skin, artificial blood vessel, artificial bone, artificial liver or the like.

When the artificial tissue of the invention is to be formed as an artificial skin, an artificial skin with an epidermis and a corium can be produced by seeding epidermal cells from the recipient animal on the artificial tissue sheet prepared in the described manner to thereby allow the cells to grow in situ and form an epidermis-like structure [Science 211, 1052, 1981]

## Brief Description of the Drawings

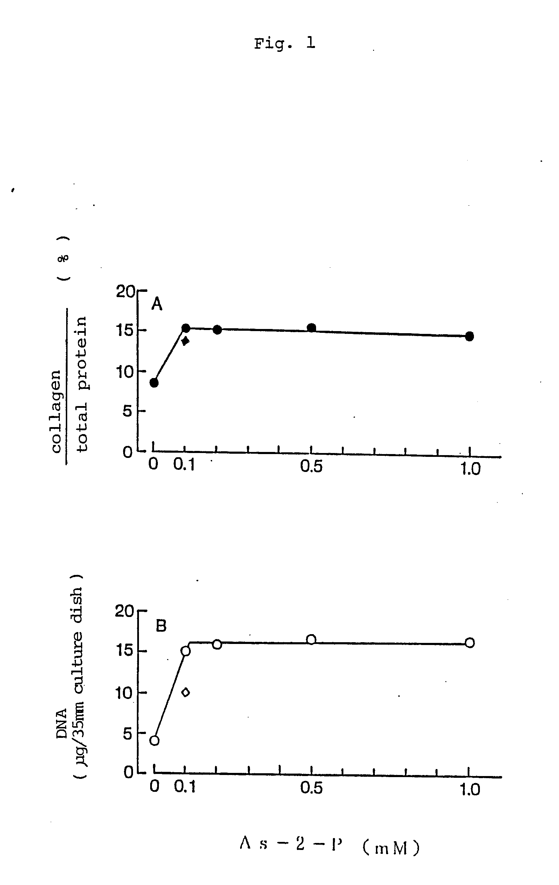

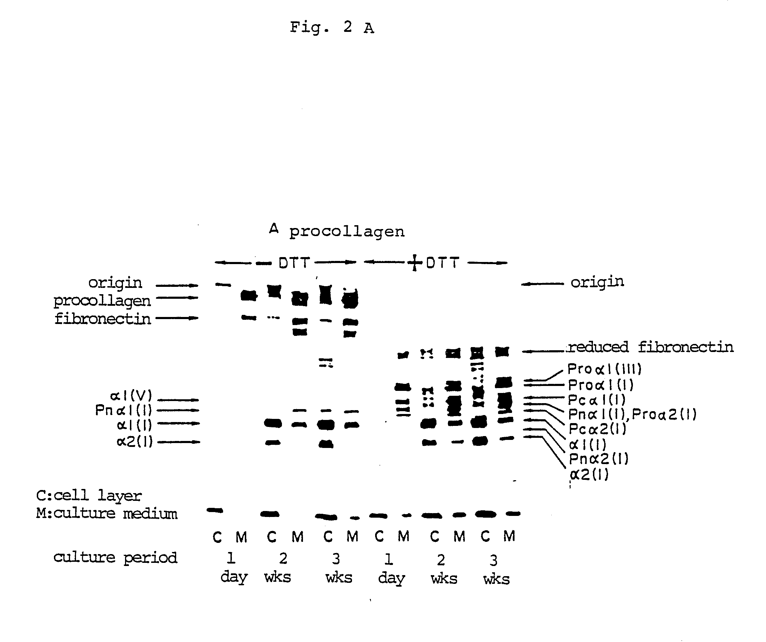

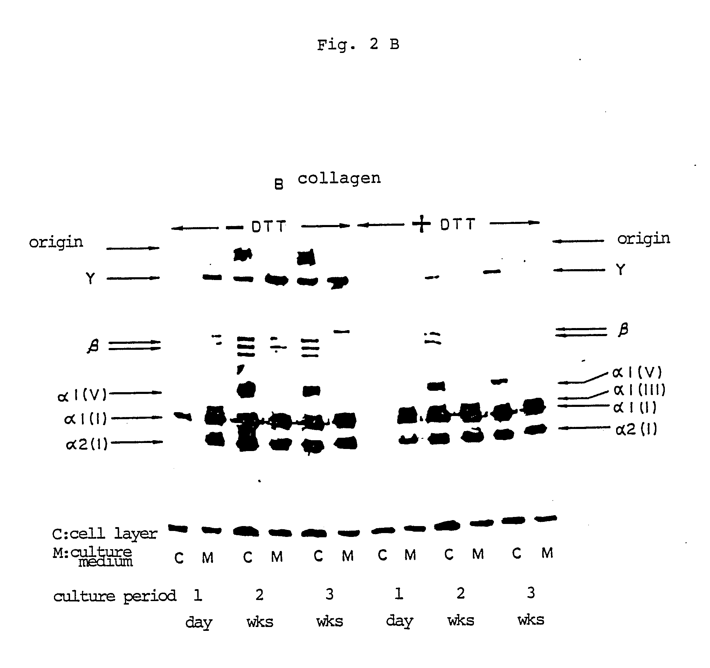

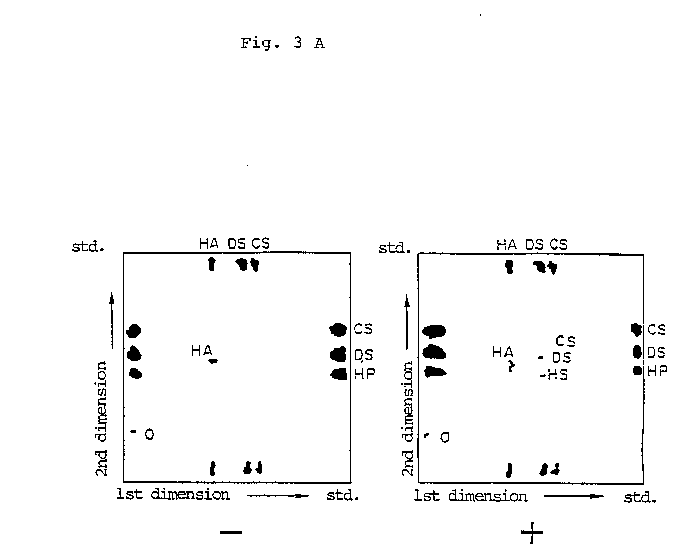

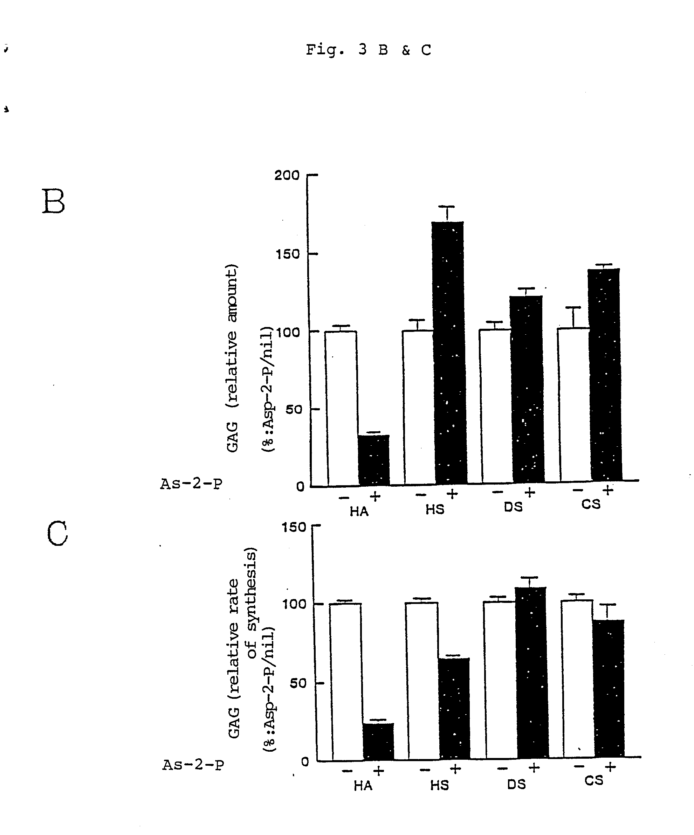

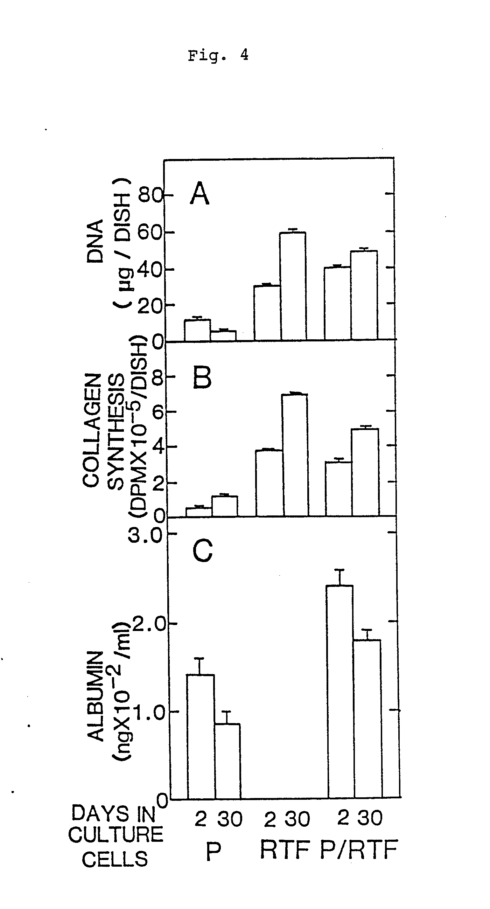

Fig. 1 A and B show the effects of accelerated proliferation of human dermatofibroblasts under the various cultural conditions described in Example 2; Fig. 2 A and B show the results of SDS-PAGE of the procollagen and collagen in the culture supernatant in Example 3; Fig. 3 A, B and C show the results of two-dimensional electrophoresis of GAG and other findings described in Example 4; and Fig. 4 A, B and C show the synthesis of DNA, collagen and albumin by rat hepatocytes, rat tendon fibroblast or by rat hepatocytes co-cultured with rat tendon fibroblasts. In the figure, P, RTF, and P/RTF show the cultures of the hepatocytes, the fibroblasts and hepatocytes co-cultured with fibroblasts, respectively.

## Examples

The following examples are further illustrative of the present invention.

### Example 1

Primary culture of human dermal fibroblasts, and subculture

An about 10 mm² patch of the skin is aseptically excised from the forearm of an adult using a surgical knife and washed with DMEM (DMEM-0) containing antibiotics (250 µl fungizone, 50 mg/l dihydrostreptomycin and 50 mg/l penicillin G). Using a razor blade, the washed skin was cut into 1 mm² squares and left on a culture dish for about 10 minutes. After addition of DMEM containing 10% (v/v) fetal calf serum (FCS), called DMEM-10 for short, the tissue was grown in a carbon dioxide gas incubator at 37°C, with the medium being changed with a fresh one every 2-3 days, until the cells cover the culture dish (primary culture). The culture medium was discarded and the cells were washed, detached from the culture dish using a trypsin solution and appropriately diluted. After addition of fresh medium, the cells were seeded again. This procedure was repeated (subculture). Hereinafter, cells after 10 to 30 serial passages were employed.

### Example 2

Promotion of cell growth and collagen synthesis in the culture of human dermal fibroblasts using the ascorbic acid 2-phosphate (As-2-P)-containing medium (Fig. 1)

The cells prepared in Example 1 were seeded on a culture dish with a diameter of 3.5 cm (Falcon, U.S.A.) and after addition of 0.1-1.0 mM of As-2-P to the above medium (●,○), culture was carried out in 5% $CO_2$-95% air at 37°C for a total of 3 weeks. As a control, several runs were performed without addition of As-2-P. The medium was replaced with a fresh medium containing a varying concentration of As-2-P with a frequency of twice a week and the culture was continued for a total of 3 weeks. In some runs (    ,◆), the culture was carried out without addition of As-2-P during the first one week and, then, in the presence of 0.1 mM of As-2-P during the subsequent two weeks. During the last 24-hours in the 3-week culture period, the culture was conducted in DMEM-10 containing ³H-proline (10 μCi/ml) and As-2-P (0.1 mM), and the culture supernatant was combined with the cells. Thereafter, in accordance with the method of Hata et al. [Biochemistry 19 169 (1980)], the rate of collagen synthesis was determined from the uptake of ³H-proline digested by a non-specific protease-free collagenase of bacterial origin [Biochemistry 10, 988 (1971)] (Fig. 1 A)

Aside from the above, after 3 weeks of culture, the supernatant was discarded and the cell layer was washed with 1 ml of Tris buffered saline (0.05 M Tris-HCl, 0.11 M NaCl, pH 7.4). After addition of 1 ml of cold trichloroacetic acid (10%), the cells were collected and the DNA content was determined by fluorometry in accordance with the method of Hata et al. [Journal of Cellular Physiology 122, 333 (1985)] (Fig. 1 B).

It is apparent from Fig. 1 A that compared with the culture conducted in the presence of As-2-P only during the last day, the culture conducted in the presence of As-2-P throughout the whole 3-week period resulted in a two-fold increase in the rate of collagen synthesis. When sodium ascorbate (AsNa) was added, activation of collagen synthesis was less effective at 0.2 mM and higher concentrations. With As-2-P, however, no inhibition was found at all, even at the level of addition of 1 mM.

It will be apparent from Fig. 1 B that the 3-week culture in the presence of As-2-P resulted in a 4-fold increase in DNA content per culture dish. Moreover, the finding that even the addition of As-2-P only during the last 2-week period caused an increase in DNA content suggests that As-2-P not only exerts a promoting effect on the stage of cell attachment but also promotes cell proliferation.

Such effects of As-2-P were found not only in the dermal fibroblasts of healthy humans but also in the dermal fibroblasts of patients with various connective tissue diseases and the pulmonary fibroblasts of fetuses.

## Example 3

The procollagen and collagen produced by culture of human dermal fibroblasts using the As-2-P-containing medium (Fig. 2)

The cells prepared in Example 1 were cultured in the same manner as Example 2 (except that the concentration of As-2-P was 0.1 mM) and labeled with ³H-proline. After the culture, the supernatant (M) and cell layer (C) were independently collected and discrupted by sonication on an ice-bath. To each of the above fractions, ammonium sulfate (176 mg/ml) was added in the presence of protease inhibitors (25 mM EDTA, 10 mM N-ethylmaleimide, 1 mM phenylmethylsulfonyl fluoride) and the resulting precipitate was dissolved in 0.5 M acetic acid. To one-half volume of the solution was added protease inhibitors (containing 200 μg each of pepstatin, antipain and leupeptin in 5 ml), followed by addition of 5 mM acetic acid. The mixture was dialyzed to obtain a procollagen fraction.

The remaining half of the above acetic acid solution was treated with 100 μg/ml of pepsin at 5°C for 6 hours, at the end of which time the pepsin was inactivated by increasing the pH to 8. After addition of pepstatin, the mixture was dialyzed to give a collagen fraction.

Lyophilized procollagen and collagen samples were separated by SDS-5% PAGE in the presence or absence of dithiothreitol (DTT) in accordance with the method of Hata et al. [Biochemistry 19, 169 (1980); Journal of Cellular Physiology 122, 333 (1985)]. Referring to Fig. 2, the following types of collagen were detected. α1(I), α2(I), α1(III) and α1-(V): α1 and α2 represent types of α-chain constituting the collagen and I to V represent types of collagen. Pro, Pn and Pc represents procollagen, Pn collagen and Pc collagen. β or γ means that the crosslinking had occurred between 2 or 3 α-chains.

As shown in Fig. 2 A, the culture in the presence of As-2-P resulted in an approximately 10-fold increase in collagen-like substances and the promotion of processing from procollagen to collagen (particularly pronounced in the cell layer and in types I and V of collagen) was observed. Furthermore, in the cell layer, presence of β and γ components which are products of cross-linking of α-chains of collagen were observed. These crosslinks were found even after reduction with DTT and the components disappeared on treatment with purified collagenase.

The foregoing effects arising from the addition of As-2-P are most akin to those found in the biological tissue and were not found when the culture was conducted without addition of As-2-P.

## Example 4

The GAG produced by cultures of human dermal fibroblasts using the As-2-P-containing medium (Fig. 3).

The cells prepared in Example 1 were cultured and labeled in the same manner as Example 2. However, ³H-glucosamine (20 μCi/ml) was used instead of ³H-proline. Crude GAG was prepared by treatment with Actinase E [Kaken Seiyaku K.K.] in accordance with the method of Hata et al. [Biochemica Biophysica Acta 304, 408 (1973)].

This GAG was subjected to two-dimensional electrophoresis (first dimension: 0.1 M pyridine-0.47 M formic acid, pH 3; second dimension: 0.1 M barium acetate) on a cellulose acetate membrane and stained with Alcian blue (Fig. 3A). The legends in the diagram have the following meanings. HA: hyaluronic acid, DS: dermathane sulfate, CS: chondroitin-4 and/or-6 sulfate, HS: heparan sulfate, HP: heparin.

After the two-dimensional electrophoresis, the amount of bound dye to each stained GAG spot was colorimetrically determined and the amount per cell (nM hexosamine/$\mu$g DNA) was calculated for each GAG and the results were compared with the value obtained by culture in the absence of As-2-P being taken as 100 (Fig. 3 B).

After the two-dimensional electrophoresis, each GAG spot was cut out, dissolved in 1 ml of dioxane and determined for radioactivity. Then, the rate of GAG synthesis was calculated and compared with the value found without addition of As-2-P being taken as 100 (Fig. 3C).

As apparent from the diagram, the analysis for GAG, which is a main constituent of ECM other than collagen, revealed that the culture in the presence of As-2-P had resulted in an increased accumulation of sulfated GAG which is found in large amounts in biological tissues. It was also found that this increase was not brought about by acceleration of synthesis but by suppression of degradation of GAG. Since collagen is known to influence the metabolism of GAG, the above change in GAG metabolism appears to be correlated with the above-mentioned promotion of collagen synthesis.

Aside from the above study, when human dermal fibroblasts were cultured in the As-2-P-containing medium in the same manner as Examples 2 through 4 and observed electron-microscopically, the development of rough endoplasmic reticulum and Golgi apparatus was confirmed. In addition, an extensive deposition of a proteoglycan, which was a GAG-protein complex, and collagen fiber around the cells.

Moreover, a multi-layered cell architecture apparently attributable to promotion of cell growth and development of ECM was clearly observed.

These changes found in human dermal fibroblasts in culture with the addition of As-2-P as demonstrated in Examples 2 through 4, i.e. promotion of cell prolifera tion, promotion of synthesis of collagen-like substances, stimulation of processing from procollagen to collagen, promotion of crosslinking between collagen molecules, synthesis and accumulation of proteoglycans which are found in large amounts in biological tissues and of collagen in the cell layer as well as electron-microscopic findings, are all endorsing our assertion that the addition of As-2-P to the culture system of dermal fibroblasts promotes the formation of a tissue resembling the biological tissue.

By co-culturing a connective tissue cell with cell of other organ than skin, e.g. liver cell, under the present cultivating condition, said tissue resembling the biological tissue may be produced.

Example 5

Cell isolation and culture

Hepatocytes were isolated from adult male rats of the Wistar strain (body weight, 230-270 g) by the two-step perfusion method as described in J. Cell. Physiol., 122, 333 (1985). The cells (2.5 $\times$ 10$^5$) were plated onto 35-mm plastic culture dishes (ca. 10 cm$^2$) or onto fibroblasts precultured on the dishes and cultured in Williams' medium E supplemented with 10% fetal bovine serum, 10$^{-6}$M insulin, 10$^{-5}$M dexamethasone, 2 $\times$ 10$^{-4}$M As-2-P, 10$^{-2}$M N-2-hydroxyethyl-piperazine-N'-2-ethanesulfonic acid, Fungizone (0.25 mg/l), penicillin (50 mg/l), and streptomycin (50 mg/l) under 5% CO$_2$/95% air at 37°C. The medium was replaced one day and 2 days after inoculation and then twice a week thereafter. EGF (20 ng/ml) was added at the time of refeeding.

Tendon fibroblasts were isolated from rat Achilles tendon by treatment with collagenase and trypsin. The cells were plated onto 35-mm dishes and cultured in Dulbecco's modified Eagle's medium [GIBCO] supplemented with 10% fetal bovine serum, 2$\times$10$^{-4}$M As-2-P, and antibiotics for one week then cultured with Williams' medium E with the supplements as described above in the presence or absence of hepatocytes.

Metabolic labeling and collagen assay

After 2 or 30 days in culture, the medium was replaced with 1 ml of fresh Williams' medium E with the supplements and 10 $\mu$Ci of [$^3$H]proline was added. After a 24-hr incubation, the combined cell and medium fractions were sonicated on ice and processed for the determination of collagen and noncollagenous protein syntheses using collagenase free from non-specific proteases. Relative rate of collagen synthesis was calculated assuming that collagen has an imino acid content 5.4 times higher than that of other proteins [Biochemistry, 19, 169 (1980)].

Quantitation of DNA

After removal of the medium, the cell layer was rinsed with 1 ml of 0.05 M Tris-HCl buffer (pH 7.4) containing 0.11 M NaCl and processed for the measurement of DNA content by a modification of a fluorometric method [J. Biol. Chem., 233, 184 (1958)].

Measurement of serum albumin

Used media and sonicates of the cell layer were centrifuged at 1,000 $\times$ g for 5 min and the supernatant was used for the determination of albumin by radioimmunoassay as described in [Biochemistry, 19, 169 (1980)].

Metabolic activity of hepatocytes and fibroblasts in culture

Hepatocytes cultured for 2 days in Williams' medium E supplemented with dexamethasone, insulin, As-2-P, EGF, and 10% fetal bovine serum produced albumin and a low but significant amount of collagen (0.32 ± 0.02% of total protein synthesized) (Fig. 4B and C). After 30 days in culture, 50% of the DNA and 60% of the level of albumin to those of 2-day cultures were preserved (Fig. 4A and C) The synthesis of total protein by the cells was decreased but that of collagen was increased (Fig. 4B), thus the relative rate of collagen synthesis to that of total protein synthesis was 8 times increased to 2.58 ± 0.04% after 30 days in culture.

Tendon fibroblasts cultured under the same conditions synthesized collagen actively; the relative rate of collagen synthesis to that of total protein was 6.14 ± 0.10% after 2 days in Williams' medium E with the supplements. The cells grew well during 4 weeks in culture. The collagen synthesis (Fig. 4B) as well as total protein synthesis per dish was increased at that time, but the relative rate dropped to 3.95 ± 0.11% after 30 days in culture. This lower rate of collagen synthesis after 30 days may have been due to the presence of EGF in the culture medium because EGF attenuates the stimulatory effect of ascorbate on collagen synthesis by fibroblasts.

When hepatocytes and fibroblasts were co-cultured for 2 days, the cell number determined by DNA content was the same as the sum of the two kinds of cells cultured separately. The collagen synthesis determined as collagenase-sensitive radioactivity per dish was lower than that of the fibroblasts cultured alone, even though the total cell number was higher in the co-culture. And the level of albumin was higher in the co-culture than in the case of hepatocytes cultured alone, even though fibroblasts did not produce any amount of albumin (Fig. 4B and C), indicating the presence of metabolic interactions between the two kinds of cells. After 30 days in culture, the cell number as well as collagen synthesis was increased, but the values were less than those of fibroblasts cultured alone. The level of albumin was 75% of that of 2-day cultures, and but still higher than the maximum level attained by the hepatocytes cultured on plastic substratum (Fig. 4C), again indicating the interactions between hepatocytes and fibroblasts. -

The addition of 0.2 mM As-2-P, a long-acting vitamin C derivative, improved the longevity of cultures and maintenance of long-term functional activity of adult hepatocytes as judged by the albumin level. About half of the albumin was present in the culture medium and the remainder in the cells. When the level of albumin in the spent media was measured during the culture period, a similar level of albumin was always detected. This fact indicated that albumin present in the medium was mainly due to production and secretion by the hepatocytes and not to lysis of the cells. In the absence of As-2-P, over 95% of the hepatocytes were lost during 30 days in culture. These results suggest that even though Williams' medium E used in the culture contains ascorbic acid, the free ascorbic acid in it is very unstable in solution, especially under culture conditions of neutral pH and 37°C, and may be degraded easily.

## Claims

1. A method of producing an artificial tissue which comprises growing a connective tissue cell in an ascorbic acid phosphate-containing medium to produce a tissue comprising connective tissue cells and extracellular matrix and recovering the same.

2. The method according to claim 1, wherein the connective issue cell is fibroblasts, smooth muscle cell, chondrocyte or endothelial cells.

3. The method according to claim 2, wherein the fibroblast is human dermal fibroblasts.

4. The method according to claim 1, wherein the ascorbic acid phosphate is ascorbic acid 2-phosphate, ascorbic acid 3-phosphate, ascorbic acid 2,3-diphosphate or a mixture of these two or three esters.

5. The method according to claim 4, wherein the ascorbic acid phosphate is ascorbic acid 2-phosphate.

6. The method according to claim 1, wherein the medium contains the ascorbic acid phosphate in a concentration of 0.01 mM to 5 mM.

7. The method according to claim 1, wherein the medium is a medium for animal cell culture containing animal serum.

8. The method according to claim 7, wherein the medium further contains an antibiotic.

9. The method according to claim 1, wherein the growing is conducted at a temperature of about 30 to 40°C and at a pH of about 6.5 to 8.

10. The method according to claim 1, wherein the growing is conducted for 2 weeks or longer.

11. The method according to claim 1, wherein the resulting tissue is in a form of a sheet.

12. The method according to claim 11, wherein the tissue is an artificial skin.

13. The method according to claim 1, wherein the resulting tissue is essentially consisting of collagen, procollagen and acidic glcosaminoglycan.

14. The method according to claim 1, wherein the connective tissue cell is co-cultivated with a liver cell to produce an artificial liver tissue.

15. An artificial tissue comprising connective tissue cells and extracellular matrix produced by growing a connective tissue cell in an ascorbic acid phosphate-containing medium.

0 282 746

Fig. 1

Fig. 2 A

Fig. 2 B

B collagen

Fig. 3 A

Fig. 3 B & C

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | PROC. NATL. ACAD. SCI. USA, vol. 53, 1965, pages 335-342; B. PETERKOFSKY et al.: "Enzymatic hydroxylation of proline in microsomal polypeptide leading to formation of collagen" | | C 12 N 5/00 |
| A,D | JOURNAL OF CELL BIOLOGY, vol. 92, February 1982, pages 462-470, The Rockefeller University Press; E. SCHWARTZ et al.: "Changes in the components of extracellular matrix and in growth properties of cultured aortic smooth muscle cells upon ascorbate feeding" | | |
| A | CHEMICAL ABSTRACTS, vol. 103, no. 26, 30th December 1985, page 352, no. 220741c, Columbus, Ohio, US; H. PADH et al.: "Stability of ascorbic acid in solution" & IRCS MED. SCI. 1985, 13(10), 1028-9 | | |
| A | WO-A-8 404 325 (INSTITUT NATIONAL DE LA SANTE ET DE LA RECHERCHE MEDICALE) | | TECHNICAL FIELDS SEARCHED (Int. Cl.4) C 12 N |
| P,X | PATENT ABSTRACTS OF JAPAN, vol. 11, no. 242 (C-348)[2689], 7th August 1987; & JP-A-62 48 376 (TAKEDA CHEM IND LTD) 03-03-1987 | 1-15 | |

The present search report has been drawn up for all claims

| Place of search | Date of completion of the search | Examiner |
|---|---|---|
| THE HAGUE | 17-05-1988 | ALVAREZ Y ALVAREZ C. |